Europäisches Patentamt

European Patent Office

Office européen des brevets

(11) Veröffentlichungsnummer : **0 049 379**
**B1**

(12) **EUROPÄISCHE PATENTSCHRIFT**

(45) Veröffentlichungstag der Patentschrift :
18.01.84

(21) Anmeldenummer : 81107125.7

(22) Anmeldetag : 10.09.81

(51) Int. Cl.³ : **A 61 K 35/407**, C 07 G   7/00,
C 07 C  15/00

(54) Verfahren zur Herstellung eines die Proliferationsrate von Leberzellen stimulierenden Faktors.

(30) Priorität : 04.10.80 DE 3037600

(43) Veröffentlichungstag der Anmeldung :
14.04.82 Patentblatt 82/15

(45) Bekanntmachung des Hinweises auf die Patenterteilung : 18.01.84 Patentblatt 84/03

(84) Benannte Vertragsstaaten :
CH FR GB IT LI NL SE

(56) Entgegenhaltungen :
EP-A- 0 004 668
EP-A- 0 017 867
Chemical Abstracts Band 92, Nr. 25, 23. Juni 1980
Columbus, Ohio, USA M. GOLDBERG et al. "Evidence
for and characterization of a liver cell proliferation
factor from blood plasma of partially hepatectomized
rats" Seite 396, Spalte 2, Abstract Nr. 212730n

(73) Patentinhaber : **Max-Planck-Gesellschaft zur Förderung der Wissenschaften e.V.
Bunsenstrasse 10
D-3400 Göttingen (DE)**

(72) Erfinder : **Ruhenstroth-Bauer, Gerhard, Prof. Dr.
Spitzelbergerstrasse 11
D-8032 Gräfelfing (DE)**
Erfinder : **Goldberg, Michel, Dr.
Hohenzollernplatz
D-8000 München 40 (DE)**
Erfinder : **Schneider, Hubertus, Dr.
Romanstrasse 100
D-8000 München 19 (DE)**

(74) Vertreter : **Dreiss, Uwe, Dr. jur. Dipl.-Ing. M.Sc. et al
Patentanwälte Dreiss, Hosenthien & Fuhlendorf
Gerokstrasse 6
D-7000 Stuttgart 1 (DE)**

## Verfahren zur Herstellung eines die Proliferationsrate von Leberzellen stimulierenden Faktors

In dem deutschen Patent 28 14 981 (siehe auch EP-A-0 004 668) wird ein Faktor zur Stimulation der Proliferationsrate von Leberzellen beschrieben, der durch ein Neuraminsäurefreies Protein oder Proteid mit einem Molekulargewicht von 30 000 bis 50 000 D gebildet wird. Man erhält diesen Faktor dadurch, daß von teilhepatektomierten Versuchstieren die Restlebern homogenisiert, auf einen pH-Wert von 5,5 gebracht, bei 95 °C hitzedenaturiert und zentrifugiert werden, wobei der Überstand den Faktor enthält. Alternativ hierzu kann man anstelle der Restlebern teilhepatektomierter Versuchstiere das Blutplasma teilhepatektomierter Versuchstiere einsetzen (EP-A-0 017 867). Man erhält dann jedoch nicht den Faktor selbst, sondern einen Vorfaktor, der durch eine Behandlung mit Neuraminidase noch Neuraminsäure-frei gemacht werden muß.

In Anlehnung an die bestehende wissenschaftliche Nomenklatur wurde für diesen Faktor, der ein Hormon darstellt, der Name « Hepatopoetin » vorgeschlagen.

Die Bildung dieses Hormons führte zu der folgenden Hypothese über ein Regelungsmodell : die teilhepatektomierte Leber (d. h. eine Leber, von der ein Teil operativ entfernt worden ist) sendet einen Reiz an ein zunächst noch unbekanntes Organ, das daraufhin den genannten Vorfaktor produziert und in das Blutplasma abgibt. Durch eine Umsetzung mit Neuraminidase entsteht aus dem Vorfaktor der eigentliche Faktor (Hepatopoetin), der in der Leber selbst zu einer erhöhten Teilungsaktivität der Leberzellen führt.

Die in dem genannten Patent beschriebene Gewinnung des Hepatopoetins hat den Nachteil, daß als Ausgangsmaterial die Restlebern oder das Blutplasma teilhepatektomierter Tiere bereitgestellt werden müssen. Das heißt, zunächst müssen den Tieren in relativ komplizierten Operationen Teilstücke der Leber entfernt werden ; dann muß eine bestimmte Zeit lang gewartet werden, damit durch den vermuteten Regelkreis induzierte Anreicherung des Faktors in der Leber und im Plasma eintreten kann. Dann müssen die Tiere getötet, die Restlebern entnommen und der genannte Herstellungsprozeß durchgeführt werden.

Aus dieser Situation heraus ergibt sich die Aufgabe, nach weiteren Möglichkeiten der Herstellung zu suchen, insbesondere einer Art der Herstellung aus dem das Hepatopoetin selbst erzeugenden Organ. Dieses Organ war aber seither nicht bekannt.

Die genannte Aufgabe wird dadurch gelöst, daß man die Peyer'schen Drüsen von Versuchstieren homogenisiert, auf einen pH-Wert von 5,5 bringt, bei 95 °C hitzedenaturiert und zentrifugiert, wobei der Überstand den Faktor enthält.

Diese Lösung basiert auf der Erkenntnis, daß das Hepatopoetin im Körper von Säugetieren in den sog. Peyer'schen Drüsen (Peyer'sche Plaques) gebildet wird. Dies sind Lymphfollikel (Ansammlungen von Lymphozyten) am terminalen Ileum (Dünndarm) vor seiner Einmündung in das Colon (Dickdarm).

Zunächst ergab sich, daß ein aus den Peyer'schen Drüsen teilhepatektomierter Tiere gewonner Extrakt in gleicher Weise wie der nach dem in der DE-PS 28 14 981 beschriebenen Verfahren gewonnene Extrakt auf die Proliferationsrate der Leberzellen stimulierend wirkt. Gleichzeitig ergab sich jedoch — und das ist ein Verfahren mit erheblichen Vorteilen aus den o. g. Gründen —, daß auch ein aus den Peyer'schen Drüsen normaler (also nicht teilhepatektomierter) Tiere gewonnener Extrakt prinzipiell dieselbe proliferationsfördernde Wirkung hat. Dabei wurde der Extrakt auf dieselbe Art und Weise gewonnen, wie das in der DE-PS 28 14 981 beschrieben ist ; d. h., die Homogenate der operativ isolierten Peyer'schen Drüsen wurden auf einen pH-Wert von 5,5 gebracht, bei 95 °C hitzedenaturiert und zentrifugiert, wobei der Überstand das Hepatopoetin enthält.

Diese proliferationsfördernde Wirkung des aus den Peyer'schen Drüsen gewonnenen Extraktes ist sowohl ohne, als auch, und dies dann in verstärktem Maße, mit anschließender Behandlung durch Neuraminidase gegeben. Es hat deshalb den Anschein, daß durch Extrahierung aus den Peyer'schen Drüsen sowohl der in EP-A-0 017 867 beschriebene Vorfaktor (der durch anschließende Behandlung mit Neuraminidase den Faktor ergibt), als auch der eigentliche Faktor selbst gewonnen wird.

Die gewerbliche Verwertbarkeit ist durch die Verwendung dieses Faktors beim Menschen gegeben. Klinische Versuche beim Menschen sind jedoch in absehbarer Zeit nicht möglich. Doch ist dadurch die gewerbliche Verwertbarkeit nicht in Frage gestellt, denn, wie aus den folgenden Darlegungen ersichtlich, ist es allgemeines Wissen des Fachmannes auf diesem Gebiet, daß auf diese Art gewonnene Proteine oder Proteide nicht speziesspezifisch sind. Zum Nachweis wird auf folgende Veröffentlichung Bezug genommen :

a) Iscove, Sieber u. Winterhalter, Erythroid Colony Formation in Cultures of Mouse and Human Bone Marrow : Analysis of the Requirement for Erythropoietin by Gel Filtration and Affinity on Agarose-Concanavalin A, J. Cell. Physiol 83, 309-320.

In dieser Veröffentlichung wird die Wirksamkeit des aus dem Blutplasma von Schafen gewonnenen Erythropoietins auf die menschliche Knochenmarkzellen beschrieben.

b) Korner u. Hogan, The Effect of Growth Hormone on Inducible Liver Enzyms, in : Pecile u. Müller (Hrsg.), Growth and Growth Hormone (1972), S. 98-105.

In dieser Veröffentlichung wird die Wirkung eines vom Rind gewonnenen Wachstumshormons bei Ratten beschrieben (vgl. z. B. die Legende zu Fig. 3).

c) Schally u. Arimura, Growth Hormone-Releasing Hormone (GH-RH) of the Hypothalamus ; its Chemistry and in Vivo and in Vitro Effects, in : Pecile u. Müller (Hrsg.), Growth and Growth Hormone (1972), S. 247-251.

In dieser Veröffentlichung wird die Wirkung eines vom Schwein gewonnenen Hormons bei Ratten beschrieben.

d) Aus den Veröffentlichungen : Goldstein, Isolation of Bovine Thymin ; a Polypeptide Hormon of the Thymus, Nature, 247, 11 (1974) und Kagan u. a., Induction of Human Granulocyte Differentiation in Vitro by Ubiquitin and Thymopoietin, Blood 50, 275-288 (1977) ergibt sich die Wirkung des vom Rind gewonnenen Thymopoetins beim Menschen. Die erstgenannte Veröffentlichung betrifft die Gewinnung vom Rind ; die zweitgenannte die Austestung beim Menschen.

e) Es ist ferner allgemein bekannt, daß das bei Ratten und Mäusen Wirksamkeit entfaltende Insulin auch beim Menschen wirksam ist : Garcia, J. F. Assays for Erythropoietin, J. W. Fisher (Hrsg.), Kidney Hormones, 1977, S. 7-35.

Im folgenden wird die Bildung eines Extraktes beschrieben. Es handelt sich dabei um die Extraktion aus den Peyer'schen Drüsen teilhepatektomierter Versuchstiere.

Die Versuche wurden mit weiblichen 95 bis 105 g schweren SPF-Wistar-Ratten (Institut für Strahlen- und Umweltforschung, Neuherberg/München) durchgeführt. Die Tiere wurden getötet, entblutet und die Peyer'schen Drüsen mikrochirurgisch entfernt. Die Peyer'schen Drüsen wurden dann zusammen mit der vierfachen (Gewichts-) Menge zweifach destillierten Wassers in einem Elvehjem-Potter homogenisiert. Dabei wurde das Zellmaterial der Peyer'schen Drüsen zerkleinert und möglichst vollständig zerstört. Das genannte Gerät ist bekannt ; die Zellen werden dabei in einem Glaszylinder zwischen diesem und einem darin rotierenden Teflonstift verrieben. Die in den Zellen enthaltenen Stoffe werden so für die nachfolgenden Konzentrations- bzw. Trennungsschritte zugänglich.

Diese aus den Peyer'schen Drüsen gewonnenen Zellhomogenate werden mit Salzsäure (HCl-) Lösung in der Konzentration 0,1 N auf den pH-Wert gebracht. Diese Ansäuerung ist ein wichtiges Selektionsmittel zur Ausscheidung einer großen Anzahl von Proteinen : sie werden ausgefällt und damit dem weiteren Konzentrations- bzw. Isolationsprozeß entzogen. Es verbleiben also lediglich solche Proteine in dem Homogenat, die bei einer Ansäuerung auf pH = 5,5 noch stabil sind. D. h. : sie sind stabil bei einem numerischen Wert pH $\geqq$ 5,5 (da ein numerisch kleinerer pH-Wert einer stärkeren Ansäuerung entspricht). Anschließend werden die Homogenate bei einer Temperatur von 95 °C für die Dauer von 20 Minuten hitzedenaturiert. Dies hat den Zweck, weitere Bestandteile auszufällen, nämlich solche, die bei dieser oder höherer Temperatur nicht beständig sind. Durch diese beiden Verfahrensschritte (Ansäuerung auf pH = 5,5 und Hitzedenaturierung bei 95 °C) wird also ein großer Anteil der Bestandteile des Homogenats zerstört und damit ausgefällt. Anschliessend erfolgt eine Zentrifugierung für die Dauer von 15 Minuten mit 4 000 g (Minifuge Christ, Osterrode/Harz). Der Überstand nach der Zentrifugierung enthält somit nur diejenigen der insgesamt im ursprünglichen Homogenat enthaltenen Wirkstoffe, die bei pH = 5,5 und 95 °C noch stabil sind, diese jedoch in gereinigter Form.

Um auch noch diejenigen Teile dieser Wirkstoffe zu erfassen, die in den Niederschlägen welche man bei der Zentrifugierung erhalten hat, nach enthalten sind, wurden diese Niederschläge ihrerseits wieder mit Aqua bidest. auf das ursprüngliche Volumen aufgefüllt, erneut einer Ansäuerung auf pH = 5,5 und einer Hitzedenaturierung bei 95 °C unterzogen und zentrifugiert. Dieser Vorgang wurde insgesamt zweimal wiederholt.

Diese Überstände aus nunmehr insgesamt drei Zentrifugationsvorgängen werden zusammengegeben und lyophilisiert, d. h. unter Wasserentzug im Vakuum einer Gefriertrocknung unterzogen. Sie stehen dann in pulverisierter Form zur Verfügung und werden bei − 20° aufbewahrt. Diese Substanz bildet den bei den nachfolgend beschriebenen Versuchen verwendeten Extrakt (TH-Extrakt).

Gleichzeitig wurde auf dieselbe Art und Weise Extrakt von Tieren gewonnen, die nicht teilhepatektomiert wurden. Es handelt sich dabei also um den Extrakt aus den Peyer'schen Drüsen normaler Tiere (N-Extrakt).

Gleichzeitig wurde ein Teil des N-Extraktes mit Neuraminidase behandelt. Dazu wurde der lyophilisierte N-Überstand in Aqua bidest. gelöst, auf pH = 5,5 gebracht und eine Stunde lang bei 37 °C mit 250 U Neuraminidase-Ansatz (Behringwerke, Marburg) inkubiert. Anschließend wurde die überschüssige Neuraminidase bei 95 °C für die Dauer von 30 Minuten inaktiviert, zentrifugiert und der Überstand erneut lyophilisiert. Das Lyophilisat bildete den mit Neuraminidase behandelten Extrakt der Peyer'schen Drüsen normaler (d. h. nicht teilhepatektomierter) Tiere, den NND-Extrakt.

Es zeigte sich nun, daß alle Extrakte, also der TH-Extrakt aus den Peyer'schen Drüsen teilhepatektomierter Tiere ohne anschließende Neuraminidase-Behandlung, der N-Extrakt aus den Peyer'schen Drüsen normaler Tiere ohne anschließende Neuraminidase-Behandlung, sowie auch der NND-Extrakt aus den Peyer'schen Drüsen normaler Tiere mit anschließender Neuraminidase-Behandlung zu einer Erhöhung der Proliferation der Leberzellen führten.

Dazu wurde jeweils 1 g des betreffenden Extraktes in 0,9 %-NaCl-Lösung gelöst und eine Menge von 2,0 ml der Lösung normalen Ratten intraperitoneal (i. p.) injiziert.

Dieselbe Menge einer 0,9 %-igen NaCl-Lösung wurde Kontrolltieren ebenfalls intraperitoneal injiziert.

Die Messung der Proliferation der Leberzellen nach Injektion des TH-Extraktes erfolgte nun durch Messung der DNA-Synthese. Diese Messung kann dadurch erfolgen, daß die Menge von spezifisch in die DNA eingebauten radioaktiven Substanzen gemessen wird. Als eine solche Substanz wurde $^3$H-Methylthymidin verwendet. Dies hat eine spezielle Aktivität von 25 Ci/mmol (Hersteller : Radiochem. Center, Amersham).

Sowohl den Versuchstieren als auch den Kontrolltieren wurden 19 Stunden nach der Injektion der Extrakte TH, N bzw. NND je 50 μ Ci[3]H-Methylthymidin injiziert. Nach einer Stunde wurden die Tiere getötet. Die Leber wurde entfernt und bei − 20 °C aufbewahrt.

Danach erfolgte die Extraktion der DNA der Leber nach Weinbren und Woodward (Br. J. Exp. Path. 45, 442-449) 1964. Ein Teil dieses Extraktes wurde zu einer Radioaktivitätsmessung verwendet. Dazu wurden 1,5 ml der PCA (Perchloressigsäure)-haltigen Lösung mit 0,5 NaOH in der Konzentration 1N neutralisiert. Die entstehende Lösung wurde in einem Scintilationsgläschen mit 5 ml Triton × 100 und 10 ml Toluol (0,6 PPO ; PPO = 1,5-Diphenyloxazol). Mit Hilfe eines Liquid-Scintilationszählers (Intertechnique, Paris) wurde dann die Radioaktivität als Anzahl der Zerfälle/Minute (Zpm) gemessen. Ein weiterer Teil des DNA-Extraktes wurde zur Messung der DNA-Konzentration nach Burton (Biochem. J. 62, 315-323) 1956 verwendet. Man erhält also auf diese Weise die spezifische Aktivität in Zerfälle/Minute/Mikrogramm DNA (Zpm/μg DNA).

Es ergaben sich dabei die in der folgenden Tabelle angegebenen Werte :

### Tabelle 1

| Injiziert mit : | TH-Extrakt (Extrakt aus den Peyer'schen Drüsen teilhepatektomierter Versuchstiere) | N-Extrakt (Extrakt aus den Peyer'schen Drüsen normaler Versuchstiere) | NND-Extrakt (Extrakt aus den Peyer'schen Drüsen normaler Versuchstiere ; der Extrakt mit Neuraminidase behandelt) | Kontrolle (NaCl-Lösung) |
|---|---|---|---|---|
| DNA-Synthese (Zpm/μg DNA) | 348 ± 63 | 255 ± 74 | 329 ± 59 | 104 ± 22 |
| | n = 4 | n = 4 | n = 4 | n = 12 |

Damit ist nachgewiesen, daß die i. p.-Injetion der genannten Extrakte bei normalen Tieren zu einer erheblichen Steigerung der DNA-Synthese führt, die ihrerseits eine notwendige Voraussetzung einer Zellteilung und damit eines Leberwachstums durch Zellteilung ist.

Dabei ist von besonderer Bedeutung, daß auch eine Wirkung mit dem N- und dem NND-Extrakt erzielt werden konnte, d. h. mit einem Extrakt aus den Peyer'schen Drüsen normaler Versuchstiere. Das bedeutet, daß zur Gewinnung des Hepatopoetins eine Teilhepatektomie der Tiere, aus denen es gewonnen wird, nicht mehr erforderlich ist. Damit ist die Herstellung ganz erheblich vereinfacht worden und es eröffnen sich damit die Möglichkeiten, aus Schlachttieren die Peyer'schen Drüsen operativ zu isolieren und daraus das Hepatopoetin zu gewinnen. Das wiederum bedeutet eine erhebliche Vereinfachung in der Herstellung.

Zur Erhärtung der Tatsache, daß die Peyer'schen Drüsen für die Proliferation der Leberzellen die geschilderte Bedeutung haben, wurden noch folgende Experimente durchgeführt :

Im Abdominalbereich wurden segmentweise verschiedene Organe entfernt. Bei denselben Tieren wurde anschließend eine Teilhepatektomie vorgenommen und die Reaktion hierauf gemessen.

Hierzu wurden an 200 g schweren weiblichen SPF-Wistar-Ratten folgende Resektionen und zusätzlich eine 70 %-ige Teilhepatektomie durchgeführt :

a) Ileotransversotomie (Schein-Operation)

Nach einer Dissektion des distalen Ileums, ca. 4 cm vor der Einmündung ins Colon, erfolgte ein blinder Verschluß des distalen Ileumstumpfes. Anschließend erfolgte eine Ileocolostomie des oralen Ileumstumpfes ca. 2 cm aboral des Ileozökalwinkels mittels seromuskulärer Nahttechnik. (In Tab. 2 : Ileo).

b) Partielle Ileo-Colektomie

Zunächst wurde der Ileozökalwinkel dargestellt. Nach sorgfältiger Gefäßligatur erfolgte danach eine Resektion von ca. 4 cm distalem Ileum und ca. 2 cm proximalem Colon. Anschließend wurde eine End-zu-End-Anastomosierung des Ileumstumpfes mit dem Colonstumpf mittels seromuskulärer Einstülpnaht durchgeführt. Auf diese Weise ist also der Teil des Ileums, der die Peyer'schen Drüsen enthält, entfernt (im folgenden : Il-Co).

## c) Resektion der Peyer'schen Drüsen

Zunächst wurde der gesamte Dünndarm dargestellt. Nach Aufsuchen der Peyer'schen Drüsen erfolgte vom Ileozökalwinkel ausgehend oralwärts eine keilförmige Resektion und End-zu-End-Vereinigung des Darms mittels seromuskulärer Nahttechnik. Durchschnittlich wurden 6 Areale reseziert (im folgenden : PD).

## d) Colonresektion

Nach der Colon-Darstellung wurde eine Resektion von ca. 3 cm Colon etwa 2 cm aboral des Ileozökalwinkels durchgeführt. Danach erfolgte wieder eine End-zu-End-Vereinigung der beiden Colonstümpfe (im folgenden : Co).

## e) Mesenteriallymphknoten-Extirpation

Als erstes erfolgte die Darstellung der Mesenteriallymphknotenkette, die vom Ileozökalwinkel zur radix mesenterii zieht. Anschließend wurden schrittweise die einzelnen Lymphknoten isoliert, danach der Gefäßhilus ligiert und schließlich der Lymphknoten reseziert. Hierbei wurden also den Peyer'schen Drüsen benachbarte ähnliche Lymphzellenbereiche entfernt, die Peyer'schen Drüsen selbst jedoch belassen (im folgenden : MLK).

## f) Röntgenbestrahlung

Nach Darstellung des gesamten Darms wurden die Ratten mit einer dicken Bleiplatte abgedeckt. Durch einen schmalen Spalt wurde der gleiche Darmbereich, der bei der Ileo-Colektomie reseziert wurde, hervorgezogen. Danach wurden die Ratten für 8 Minuten mit insgesamt 400 R bestrahlt. Danach erfolgte ein allschichtiger Wundverschluß (im folgenden : Rönt.).

Die Durchführung der Schein-Operation erfolgte ebenfalls zu Kontrollzwecken, um die beobachtete Wirkung als Folge der Narkose, der Kälteeinwirkung auf die Organe, eines operativen Schocks oder dgl. auszuschließen.

Um bei diesen Tieren auch die DNA-Synthese beobachten zu können, wurde ihnen 21 h nach der Operation, jeweils 50 $\mu$Ci$^3$H-Thymidin injiziert. Genau eine Stunde später wurden die Ratten getötet. Die Leber wurde entfernt und anschließend in der bereits oben geschilderten Weise die Proliferationsrate bestimmt.

Es ergaben sich dabei die aus Tabelle 2 ersichtlichen je nach Resektionsart unterschiedlichen Regenerationsantworten.

### Tabelle 2

| Behandlung | Ileo | Il-Co | PD | CO | MLK | Rönt. |
|---|---|---|---|---|---|---|
| Spez. Aktivität In Zpm/$\mu$g DNA $\bar{S} \pm SD$ | 889 ± 167 | 216 ± 116 | 130 ± 46 | 912 ± 99 | 991 ± 139 | 1 246 ± 220 |
| n | 8 | 5 | 5 | 5 | 4 | 6 |

Bezogen auf die Kontrolloperation (Ileo) ergab sich also bei der Ileo-Colektomie (Il-Co) eine auf 24 ± 11 % verminderte Regeneration, bei einer Resektion der Peyer'schen Drüsen eine Regeneration von sogar nur noch 18 ± 5 %. Bei Resektionen, die die Peyer'schen Drüsen beließen (Co, MLK) ergab sich keine verminderte Regeneration. Bei Röntgenbestrahlung ergab sich eine sogar noch etwas erhöhte Regeneration. Dies ist möglicherweise damit erklärlich, daß die Röntgenbestrahlung durch Zerstörung von Gewebe den Faktor freisetzt.

Dieser Versuch bestätigt die Bedeutung der Peyer'schen Drüsen. Daß in diesen allerdings nicht nur ein für die Bildung des Hepatopoetins oder sein Auftreten in der Leber als Regenerationsantwort entscheidender Vorgang stattfindet, sondern tatsächlich die Produktion des Hepatopoetins selbst erfolgt, ergibt sich allerdings erst aus den oben dargestellten Versuchen der Wirkung des aus diesen Peyer'schen Drüsen gewonnenen Extraktes.

An den gewonnenen Ergebnissen ist weiterhin bedeutsam, daß sowohl der Extrakt ohne anschließende Behandlung mit Neuraminidase als auch mit anschließender Behandlung durch Neuraminidase die dargestellte Erhöhung der Proliferationsrate bewirkt. Daraus ist zu folgern, daß der Extrakt sowohl den Vorfaktor (aus dem sich mit Neuraminidase der eigentliche Faktor ergibt) als auch den Faktor selbst (der Neuraminsäurefrei ist und daher durch Behandlung mit Neuraminidase nicht mehr verändert wird) enthält.

Möglicherweise führt das zu der Vorstellung, daß beide Substanzen, nämlich sowohl der Faktor selbst als auch der Vorfaktor von den Peyer'schen Drüsen produziert werden. Sie gelangen dann über die V. Portae an die Leber. Die Leber fängt ihrerseits den Faktor selbst ab, während der Vorfaktor an den Blutkreislauf abgegeben wird, so daß er im Plasma vorhanden ist und nach Einwirkung von Neuraminidase den Faktor ergibt. Dies würde erklären, daß man im systemischen Blutplasma lediglich den Vorfaktor, nicht aber den Faktor selbst findet.

**Ansprüche**

1. Verfahren zur Herstellung eines die Stimulation der Proliferationsrate von Leberzellen bewirkenden Faktors, der durch ein Neuraminsäure-freies Protein oder Proteid mit einem Molekulargewicht von 30 000 bis 50 000 D gebildet wird, dadurch gekennzeichnet, daß die Peyer'schen Drüsen von Versuchstieren homogenisiert, auf einen pH-Wert von 5,5 gebracht, bei 95 °C hitzededenaturiert und zentrifugiert werden, wobei der Überstand den Faktor enthält.

2. Verfahren nach Anspruch 1, dadurch gekennzeichnet, daß der Überstand mit Neuraminidase Neuraminsäurefrei gemacht worden ist.

3. Verfahren zur Herstellung eines die Stimulation der Proliferationsrate von Leberzellen bewirkenden Faktors, der durch ein Neuraminsäurefreies Protein oder Proteid mit einem Molekulargewicht von 30 000 bis 50 000 D gebildet wird, dadurch gekennzeichnet, daß die Peyer'schen Drüsen teilhepatektomierter Versuchstiere homogenisiert, auf einen pH-Wert von 5,5 bringt, bei 95 °C hitzedenaturiert und zentrifugiert, wobei der Überstand den Faktor enthält.

4. Verfahren nach Anspruch 3, dadurch gekennzeichnet, daß der Überstand mit Neuraminidase Neuraminsäurefrei gemacht worden ist.

**Claims**

1. A process for producing a factor for stimulating the rate of proliferation of liver cells, said factor being a neuraminic acid-free protein or proteid with a molecular weight of 30,000 to 50,000 D, characterized in that the Peyer's glands of animals are homogenized, adjusted to a pH-value of 5.5, thermally denatured at 95 °C and centrifuged, whereby the supernatant contains the factor.

2. A process in accordance to claim 1, characterized in that the supernatant is made neuraminic acide-free by neuraminidase.

3. A process for producing a factor for stimulating the rate of proliferation of liver cells, said factor being a neuraminic acid-free protein or proteid with a molecular weight of 30,000 to 50,000 D, characterized in that the Peyer's glands of partially hepatectomized animals are homogenized, adjusted to a pH-value of 5.5, thermally denatured at 95 °C and centrifuged, whereby the supernatant contains the factor.

4. A process in accordance to claim 3, characterized in that the supernatant is made neuraminic acid-free by neuraminidase.

**Revendications**

1. Procédé de préparation d'un facteur provoquant la stimulation du taux de prolifération des cellules hépatiques, formé par une protéine ou un protéide exempt d'acide neuraminique et ayant un poids moléculaire de 30 000 à 50 000 D, caractérisé par le fait que l'on homogénéise les glandes de Peyer d'animaux d'expérimentation, on les amène à une valeur de pH de 5,5, on les dénature à la chaleur à 95 °C et on les centrifuge, le surnageant contenant alors ledit facteur.

2. Procédé selon la revendication 1, caractérisé par le fait que l'on rend le surnageant exempt d'acide neuraminique, avec la neuraminidase.

3. Procédé de préparation d'un facteur provoquant la stimulation du taux de prolifération des cellules hépatiques, formé par une protéine ou un protéide exempt d'acide neuraminique et ayant un poids moléculaire de 30 000 à 50 000 D, caractérisé par le fait que l'on homogénéise les glandes de Peyer d'animaux d'expérimentation partiellement hépatectomisés, on les amène à une valeur de pH de 5,5, on les dénature à la chaleur à 95 °C et on les centrifuge, le surnageant contenant alors ledit facteur.

4. Procédé selon la revendication 3, caractérisé par le fait que l'on rend le surnageant exempt d'acide neuraminique, avec la neuraminidase.